# FASCICULE DE BREVET EUROPEEN

(11) **EP 1 146 914 B1**
(45) Date de publication et mention de la délivrance du brevet: **15.01.2003**
(21) Numéro de dépôt: 99901079.6
(22) Date de dépôt: 03.02.1999
(51) Int. Cl.: A61L 2/02

(54) **DISPOSITIF DE STERILISATION D'UNE ENCEINTE**
VORRICHTUNG ZUM STERILISIEREN EINES HOHLRAUMS
DEVICE FOR STERILIZING A CHAMBER

(43) Date de publication de la demande: 24.10.2001
(73) Titulaire: Meditecnic Inc, Carson City, Nevada 89703-4121 (US)
(72) Inventeur: ROSSELL, Jordi, CH-1092 Belmont-sur-Lausanne (CH)
(74) Mandataire: Savoye, Jean-Paul
(86) Numéro de dépôt international: IB9900188
(87) Numéro de publication internationale: WO00045859

(56) Documents cités:
- WO-A-95/35069
- WO-A-96/33819
- FR-A- 2 330 374
- FR-A- 2 715 876

## Description

La présente invention se rapporte à un dispositif de nettoyage et de stérilisation à l'intérieur d'une enceinte, comprenant une alimentation de cette enceinte en liquide de stérilisation, et des moyens pour induire, au sein de ce liquide de stérilisation, des variations de pression, d'amplitude, de fréquence et de pente desdites variations, adaptés pour engendrer de la cavitation au sein de ce liquide.

Il a été observé que la cavitation, outre ses effets indésirables bien connus dans les systèmes hydrauliques quant à l'attaque des surfaces, le bruit et la perte de contact avec le liquide, présente d'autres caractéristiques qui peuvent se révéler intéressantes dans certaines applications.

La première de ces caractéristiques est mécanique et permet de dépasser les limites de la capillarité dans un régime cavitant. Cette propriété peut donc être intéressante lorsqu'il s'agit de traiter des régions autrement inaccessibles.

Les propriétés destructrices peuvent être utilisées à bon escient en mettant à profit l'onde thermique qui, bien que ponctuelle, est néanmoins importante. Il en est de même de la réaction d'oxydation qui l'accompagne. En effet, l'implosion exothermique des bulles de vapeur nées par dépression sur un germe libère son énergie dans un temps très court et sur une surface très petite, déterminant ponctuellement une température très élevée.

C'est donc la conjonction des effets mécanique, thermique, voire chimique qui permet à la fois une meilleure mise en oeuvre d'un agent nettoyant et/ou stérilisant et une augmentation de son efficacité. La dissolution d'une substance dans une autre est ainsi grandement améliorée et permet la stérilisation d'une cavité ou d'un corps immergé en régime cavitant, ce qui ne pourrait pas être obtenu par le simple rinçage ou l'immersion prolongée avec un même agent liquide.

La cavitation apparaissant lorsque des conditions thermodynamiques connues au sein d'un liquide déterminé sont remplies, il suffit d'imprimer à l'intérieur d'une enceinte close et remplie de liquide, les variations de pressions adéquates dans leur amplitude et leur forme pour engendrer la cavitation dans ce liquide à sa température propre. L'effet de la cavitation peut s'exercer sur le liquide lui-même, sur les parois du réceptacle ou sur tout corps qui y sera immergé.

Les exigences du signal de pression sont toutefois bien particulières et difficiles à obtenir uniquement par sélection mécanique des niveaux de pression et une fréquence souhaités.

L'utilisation de la cavitation pour le nettoyage et la stérilisation a déjà fait l'objet de nombreuses applications dans le domaine médical, ou pour le nettoyage et la stérilisation de matériel médical ou paramédical. L'association des fréquences ultrasoniques et de la cavitation a également été proposée pour le nettoyage et la stérilisation. On peut citer, à titre d'exemple, le DE 39 03 648 qui est relatif à un procédé pour inactiver des virus au sein d'un liquide par la cavitation engendrée en variant les vitesses d'écoulement au sein du liquide. Ce procédé est mis en oeuvre à l'aide d'une pompe à haute pression et d'une soupape d'homogénéisation placée en aval.

Dans le EP 0 078 614, des lentilles de contact son nettoyées et désinfectées dans une solution saline au sein de laquelle on crée de la cavitation à une fréquence ultrasonique.

Un autre procédé de nettoyage et de stérilisation associant les ultrasons et la cavitation est décrit dans le EP 0 595 783 ainsi que dans le US 4 193 818. La cavitation associée aux ultrasons présente l'inconvénient de provoquer une attaque de la surface nettoyée.

On a également proposé d'utiliser la cavitation pour dévitaliser des dents, dans le EP 0 299 919, dans lequel un embout est ajusté de façon étanche sur une ouverture ménagée pour donner accès à la chambre pulpaire de la dent. Cet embout comporte un injecteur de liquide relié à une pompe d'alimentation et un conduit d'évacuation relié à une pompe d'aspiration. La pompe d'aspiration crée des bulles dans le liquide, que la pompe de pression fait imploser produisant ainsi la cavitation.

Un perfectionnement du dispositif précédent a été proposé dans le EP 0 521 119, dans lequel une trompe à eau est agencée dans l'embout ajustable de façon étanche sur l'orifice de la chambre pulpaire de la dent remplie d'eau de Javel. L'entrée de cette trompe est reliée au conduit de sortie d'une pompe à piston, sa sortie est reliée à un conduit d'évacuation et son conduit d'aspiration débouche dans la chambre pulpaire. A chaque cycle de la pompe, un mouvement de va-et-vient d'un certain volume de liquide se produit dans le conduit d'évacuation sous l'effet des alternances de compression et d'aspiration, dans une conduite reliant la pompe au conduit d'évacuation à travers la trompe à eau, engendrant des dépressions et surpressions alternées dans le liquide de la chambre pulpaire, génératrices de cavitation.

Le premier de ces dispositifs de dévitalisation nécessite deux pompes et entraîne une consommation importante de liquide, constitué par le liquide de traitement lui-même. Le second de ces dispositifs de dévitalisation utilise une pompe à piston à simple effet qui est en quelque sorte le moteur actionnant la trompe à eau engendrant les variations de pression dans la chambre pulpaire. La pompe à piston utilisée à cet effet produit une pression sinusoïdale qui, à partir de la fermeture d'une soupape à simple effet, augmente pour atteindre la pente de la sinusoïde. Or seule la pente est utile pour créer la cavitation et cette pente doit être le plus raide possible pour créer une variation aussi brusque que possible. Etant donné que la variation sinusoïdale ne suffit pas à elle seule à provoquer la cavitation recherchée, elle agit sur la pression de la chambre pulpaire par l'intermédiaire d'une masse de liquide qu'elle met en mouvement et travaillant en résonateur pour créer les brusques variations de pression nécessaires par l'intermédiaire de la trompe à eau.

Le document WO-A-9 535 069 décrit un dispositif de nettoyage et de stérilisation à l'intérieur d'une enceinte, qui induit des variations de pression avec un générateur comportant une pompe à piston entre deux réservoirs de liquide de traitement.

Le but de la présente invention est d'atteindre le même effet, mais de manière directe sans l'intermédiaire d'une trompe à eau, donc sans générateur de pression positive.

A cet effet, la présente invention a pour objet un dispositif de nettoyage et de stérilisation à l'intérieur d'une enceinte, conforme à la revendication 1.

L'utilisation d'un commutateur permet de mettre alternativement une colonne de liquide reliant ce commutateur à l'enceinte de stérilisation en communication avec deux niveaux de pression déterminés, dont l'écart correspond à l'amplitude désirée, de sorte que la pente de la variation est très importante, seules les pertes de charge dans les conduits influençant cette pente, puisque les pressions des deux niveaux sont constantes. En outre, la pression d'un seul des deux niveaux de pression doit être créée artificiellement, celle de l'autre niveau, correspondant à la pression la plus élevée, étant simplement la pression atmosphérique. Le système fonctionne donc comme un piston à ressort, qui tend constamment à être ramené dans une de ses deux positions par le ressort de rappel. Dans le cas de la présente invention, le ressort est constitué par la pression atmosphérique.

Le dessin annexé illustre, schématiquement et à titre d'exemple, une forme d'exécution du dispositif de nettoyage et de stérilisation objet de la présente invention.
La figure 1 est un schéma bloc montrant son principe;
la figure 2 est une vue d'un détail en coupe de la figure 1;
la figure 3 est une vue d'un autre détail en élévation de la figure 1;
la figure 4 est une vue selon la ligne IV-IV de la figure 3;
la figure 5 est une vue montrant le détail de la figure 3 monté sur une dent vue en coupe, selon une première utilisation;
la figure 6 est une vue montrant le détail de la figure 3 monté sur une enceinte de nettoyage et de stérilisation pour lentilles de contact;
la figure 7 est une vue en élévation d'un endoscope dont une partie est logée dans une enceinte de stérilisation du dispositif selon l'invention, spécialement adaptée à cette utilisation;
la figure 8 est une vue en élévation, partiellement en coupe d'une autre application de l'invention.

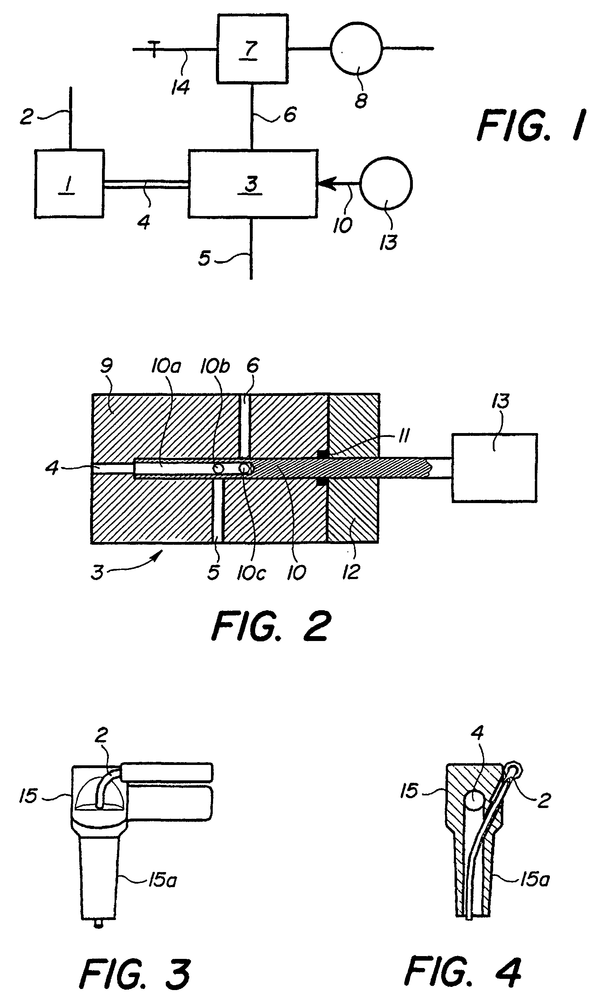

Le dispositif de nettoyage dont le principe est illustré par la figure 1 comporte une enceinte de traitement 1, reliée d'une part à une alimentation calibrée en liquide de traitement 2 et d'autre part à un organe de distribution ou de commutation 3 par l'intermédiaire d'un conduit 4. Une première entrée 5 de cet organe de commutation 3 est en communication avec la pression atmosphérique et une seconde entrée 6 est en communication avec une source de basse pression 7 reliée à une pompe à vide 8, ainsi qu'à une entrée auxiliaire d'air réglable 14. Cet organe de commutation 3, qui est représenté plus en détail par la figure 2, comporte un corps cylindrique, traversé axialement par un canal dans lequel débouchent latéralement les conduits 5 et 6 et dont une extrémité axiale communique avec le conduit 4 reliant cet organe de commutation à l'enceinte de traitement 1.

Un rotor de distribution 10 est monté dans ce canal axial, un joint O-ring 11 logé autour du rotor à une extrémité du bloc 9 et retenu par un couvercle 12, assure l'étanchéité du canal axial. L'extrémité du rotor de distribution 10 faisant saillie hors du bloc 9 est solidaire de l'arbre d'entraînement d'un moteur 13. L'extrémité du rotor 10 en communication avec le conduit 4 présente un passage axial 10a, muni de deux lumières 10b et 10c destinées à faire communiquer cycliquement le passage axial 10a, avec le conduit 5 et la pression atmosphérique, respectivement, avec le conduit 6 et la source de vide.

La première phase du processus cyclique engendré par le dispositif qui vient d'être décrit, consiste à abaisser subitement la pression dans l'enceinte de traitement 1 au-dessous de la pression de vapeur du liquide de traitement qui remplit cette enceinte 1, en la mettant en communication avec la source de basse pression 7, le rotor 10 se trouvant alors dans la position angulaire illustrée par la figure 2. La température, la nature du liquide et sa pureté auront une influence sur le niveau et la pente de la variation nécessaire. Chaque impureté ou discontinuité mécanique sera un germe potentiel de bulle pour un liquide donné. A noter que dans le cas de la présente invention, cette pente est très raide, seules les pertes de charge à travers les conduits entrant en ligne de compte pour l'établissement de la dépression dans l'enceinte 1, par contre il n'y a plus l'interférence du mouvement sinusoïdal d'une pompe comme dans les solutions de l'état de la technique.

La seconde phase de ce processus consiste à faire imploser les bulles de vapeur créées lors de la première phase, en rétablissant la pression atmosphérique dans l'enceinte 1, ce qui est obtenu par la rotation du rotor 10 qui amène la lumière 10b en communication avec le conduit 5 et la pression atmosphérique. Le liquide qui avait été aspiré dans le conduit 4 retourne alors vers l'enceinte créant une légère surpression momentanée qui provoque l'implosion simultanée de toutes le bulles de vapeur précédemment formées.

L'effet maximum du changement d'état est proportionnel au vide entretenu dans la source de basse pression 7 par une pompe à membrane 8. Le niveau de vide peut toutefois être modulé en fonction de la puissance désirée, grâce à l'entrée auxiliaire d'air réglable 14.

Le remplissage du système est assuré à travers l'enceinte de traitement 1, par l'alimentation de liquide calibrée 2, la pression moyenne en régime de fonctionnement étant négative. L'action de va-et-vient de la colonne de liquide n'est en effet assurée que pour un conduit correctement amorcé et une régénération du liquide est souhaitable même si elle ouvre le système.

La vitesse de rotation du moteur 13 entraînant le rotor 10 est ajustable en fonction de la nature du liquide utilisé, de sa température et de la dépression choisie. L'état du mélange extrait de l'enceinte de traitement 1 (proportion de gaz dissout) au travers du conduit 4 joue un rôle et peut exiger une adaptation, en particulier lors de l'opération de dissolution.

Les dimensions et la rigidité du conduit 4 reliant l'enceinte de traitement 1 au commutateur 3 sont en relation avec la fréquence du cycle. Un conduit en polyuréthane de 2mm de diamètre intérieur et 1mm de paroi pour une longueur de 320mm a donné de bons résultats à une fréquence de l'ordre de 15 à 25Hz avec la plupart des liquides utilisés. Ce dimensionnement convient pour générer un bon régime de cavitation dans des volumes allant jusqu'à quelques cm³. Les dimensions de la restriction de l'entrée de liquide frais font l'objet d'un compromis entre un bon remplissage de la tubulure et la perte inhérente de vide; un tube en acier inoxydable d'un diamètre intérieur de 0,3mm pour une longueur de 15mm a donné de bons résultats. Le débit de fonctionnement dépend du liquide utilisé et est alors de l'ordre de 10ml/min.

Le calibrage de l'entrée d'air 14 dépend de la pompe à membrane utilisée. Un robinet de réglage offre le meilleur confort d'utilisation. La pompe à membrane doit permettre d'atteindre dans la source de basse pression 7 un vide d'au moins -0,9.10⁵Pa en régime de fonctionnement lorsque l'entrée d'air 14 est complètement fermée. Il est à noter que cette pression est en elle-même plus élevée que la pression de vapeur du liquide. Toutefois, grâce à la colonne de liquide 4, il est possible d'atteindre en régime dynamique, des pics inférieurs à la valeur de la dépression de la source de basse pression 7.

Les figures 3 à 5 illustrent une application du dispositif qui vient d'être décrit utilisé pour dévitaliser une dent. Dans le cas de cette utilisation particulière, l'enceinte de traitement 1 est constituée par la chambre pulpaire P de la dent D à dévitaliser, un embout d'adaptation 15 étant destiné à relier la chambre pulpaire P de la dent D d'une part, à l'alimentation 2 en liquide de traitement, d'autre part au conduit 4 reliant la chambre pulpaire P au commutateur 3.

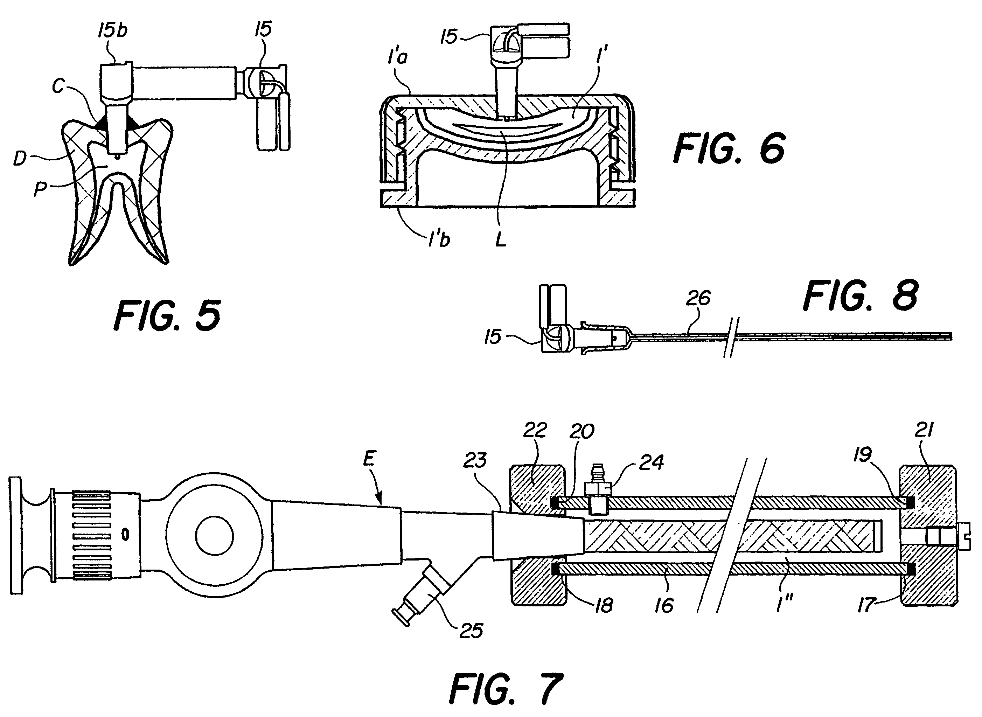

Comme le montre la figure 5, l'embout 15 comporte un élément de liaison 15a ajusté de façon étanche dans un élément de raccord flexible 15b, lui-même ajusté dans une ouverture ménagée dans la dent D pour permettre d'accéder à la chambre pulpaire P de la dent D. Un joint de fixation en ciment C formé autour de l'élément de raccord flexible 15b sert à assurer l'étanchéité de l'enceinte de traitement. Cette utilisation offre un réel avantage pour la pulpectomie des racines vitales ou pas. L'action de l'hypochlorite de sodium traditionnellement utilisée est rendue plus efficace par une interface accrue du liquide corrosif avec le nerf dentaire jusque dans les moindres recoins, même inaccessibles manuellement. L'effet stérilisant de la cavitation ajoute à l'efficacité de l'intervention, éliminant tout germe résiduel. L'opération est de plus non invasive, réduisant de ce fait le traumatisme infligé.

L'utilisation de l'élément de raccord supplémentaire offre une connexion plus ergonomique et qui est avantageusement flexible. Elle a aussi pour avantage de permettre l'enlèvement et la remise en place de l'embout 15 sans devoir casser le ciment C.

La figure 6 illustre une autre utilisation avantageuse de la présente invention pour l'hydratation et la stérilisation des lentilles de contact souples. On peut observer sur cette figure qu'un embout 15 est fixé dans une ouverture donnant accès à l'intérieur d'une enceinte de traitement 1' dans laquelle est immergée une lentille de contact souple L. L'enceinte de traitement 1' est constituée de deux parties 1'a, 1'b assemblées de manière étanche l'une à l'autre, par exemple par une fixation du type à baïonnette. Cette lentille de contact hydrophile peut être débarrassée de tout germe en faisant caviter le volume de liquide dans lequel elle reste hydratée pendant une durée de l'ordre de 10 min. Une immersion, même durant toute une nuit, dans le même produit désinfectant spécifique ne parvient pas à elle seule de venir à bout de la contamination bactérienne découlant de son utilisation.

La figure 7 illustre encore une autre utilisation avantageuse du dispositif selon l'invention, pour les appareils optiques d'endoscopie non autoclaves et plus particulièrement ceux pourvus d'un canal pour pince à biopsie. De tels appareils ne sont en effet jamais rendus stériles par la seule immersion en liquide désinfectant à laquelle ils sont soumis après chaque utilisation. La mise en cavitation et la circulation de l'agent désinfectant de rinçage dans lequel ils sont plongés les stérilise efficacement et permet leur réutilisation après un temps réduit.

L'enceinte de traitement 1" dans laquelle l'extrémité active de l'endoscope E est logée comporte un tube 16 dont les extrémités sont engagées dans deux gorges annulaires 17, respectivement 18 au fond desquelles se trouvent des joints O-ring 19, respectivement 20. La gorge annulaire 17 est ménagée dans un organe de fermeture 21, tandis que la gorge 18 est ménagée dans un anneau de fermeture 22 destiné à s'engager contre une partie tronconique 23 de l'endoscope E. Un embout 24 traverse la paroi du tube 16 et sert à relier l'intérieur du tube avec le générateur de cavitation de la figure 1. L'intérieur du tube 16 qui sert d'enceinte de traitement est alimentée en liquide de traitement à travers le canal d'accès 25 de la pince à biopsie de l'endoscope lorsqu'un tel canal existe. Sinon, une alimentation peut être prévue directement à travers la paroi du tube 16.

Une autre utilisation très voisine de celle décrite à la figure 6 pourrait être appliquée au débouchage des cathéters, qui pourrait être pratiqué sans enlèvement du cathéter. A cet effet, comme illustré par la figure 8, l'embout 15 est fixé à l'extrémité du cathéter 26 destinée à la perfusion. La cavitation se produira tant que le caillot bouche le passage, induisant un liquide anticoagulant jusqu'à l'interface du bouchon sanguin qui obstrue le conduit du cathéter 26. Elle s'arrêtera spontanément dès l'apparition d'un flux de sang frais aspiré, témoin de la réussite de l'opération, après quoi, la perfusion peut être remise à la place de l'embout 15.

L'utilisation du dispositif décrit pourrait encore s'étendre au débouchage des conduits artériels ou veineux. Toutefois, dans ce cas et compte tenu du fait que les parois de ces conduits ne sont pas rigides, il faudrait des moyens pour empêcher l'écrasement de ces conduits, étant donné que pour créer la cavitation la pression doit s'abaisser au-dessous de la pression atmosphérique

Bien entendu, le dimensionnement de la source de vide 7 et de l'organe de commutation devront être adaptés au volume nécessaire pour l'enceinte de traitement.

Il est évident que d'autres applications que celles décrites précédemment et faisant usage du même dispositif de nettoyage et de stérilisation peuvent être envisagées.

## Revendications

1. Dispositif de nettoyage et de stérilisation à l'intérieur d'une enceinte (1), comprenant une alimentation (2) de cette enceinte (1) en liquide de stérilisation, et des moyens pour induire, au sein de ce liquide de stérilisation, des variations de pression, d'amplitude, de fréquence et de pente desdites variations, adaptés pour engendrer de la cavitation au sein de ce liquide, **caractérisé en ce que** lesdits moyens pour induire des variations de pression comprennent une colonne de liquide entre ladite enceinte et un organe de commutation (3, 10) apte à relier, de manière cyclique ladite enceinte (1), à une dépression (7) dont la valeur est en relation avec ladite amplitude, respectivement la pression atmosphérique.

2. Dispositif selon la revendication 1, **caractérisé en ce qu'**il comporte un conduit principal (4) reliant ledit organe de commutation à ladite enceinte de traitement (1), deux conduits (5, 6) reliant ledit conduit principal (4) respectivement à la pression atmosphérique et à ladite dépression, ledit organe de commutation (3, 10) comprenant des passages de liaison (10b, 10c) entre lesdits conduits (5, 6) et ledit conduit principal (4) et étant mobile entre au moins deux positions l'une dans laquelle un desdits passages (10b) de liaison met le conduit principal (4) en communication avec l'atmosphère, l'autre dans laquelle l'autre desdits passages (10c) met le conduit principal en communication avec ladite dépression, et des moyens d'entraînement pour déplacer ledit organe de commutation de l'une à l'autre position et vice-versa.

3. Dispositif selon la revendication 2, **caractérisé en ce que** ledit organe de commutation est un organe rotatif et qu'il est cinématiquement solidaire de l'arbre de sortie d'un moteur d'entraînement.

4. Dispositif selon l'une des revendications précédentes, **caractérisé en ce qu'**il comporte un embout d'adaptation (15) destiné à relier ladite enceinte de traitement (1, 1') d'une part, audit organe de commutation (3, 10) et d'autre part à ladite alimentation (2) en liquide de stérilisation.

5. Dispositif selon la revendication 4, **caractérisé en ce qu'**un second élément de raccordement flexible (15) relié de façon étanche et amovible audit embout d'adaptation (15) est disposé entre ledit organe de commutation et ladite enceinte de traitement (1).

6. Dispositif selon l'une des revendications précédentes, **caractérisé en ce qu'**il comprend une enceinte de traitement (1') constituée de deux parties (1'a, 1'b) assemblées de manière amovible et étanche l'une à l'autre.

7. Dispositif selon l'une des revendications 1 à 4, **caractérisé en ce qu'**il comprend une enceinte (1'') constituée par un élément tubulaire (16) dont une extrémité est ouverte pour recevoir la partie de travail d'un endoscope (E), l'intérieur de cette enceinte (1'') étant relié d'une part audit organe de commutation (3, 10) par l'intermédiaire d'un embout de liaison (24) et d'autre part à ladite alimentation de liquide de stérilisation par le canal d'entrée (25) de la pince à biopsie dudit endoscope (E).

8. Dispositif selon la revendication 7, **caractérisé en ce que** ladite enceinte tubulaire (1") comporte un élément tubulaire (16) ouvert à ses deux extrémités, chacune d'elle étant engagée dans une gorge annulaire (17, 18) de deux organes de fermeture (21, 22), avec interposition d'un joint d'étanchéité (19, 20).

## Claims

1. Device for cleaning and sterilizing the inside of a chamber (1), comprising a supply (2) of sterilizing liquid for this chamber (1), and means for inducing, within this sterilizing liquid, variations in pressure, amplitude and frequency, and in the gradient of said variations, said means being adapted to generate cavitation within this liquid, **characterized in that** said means for inducing variations in pressure comprise a liquid column between said chamber and a switching member (3, 10) with which said chamber (1) can be connected cyclically to a negative pressure (7), the value of the latter being in relation to said amplitude or respectively to the atmospheric pressure.

2. Device according to Claim 1, **characterized in that** it comprises a main conduit (4) connecting said switching member to said treatment chamber (1), two conduits (5, 6) connecting said main conduit (4) to the atmospheric pressure and to said negative pressure, respectively, said switching member (3, 10) comprising connecting passages (10b, 10c) between said conduits (5, 6) and said main conduit (4) and being movable between at least two positions, one in which one of said connecting passages (10b) brings the main conduit (4) into communication with the atmosphere, the other in which the other of said passages (10c) brings the main conduit into communication with said negative pressure, and drive means for displacing said switching member from one position to the other and vice versa.

3. Device according to Claim 2, **characterized in that** said switching member is a rotary member and is integral in kinematic terms with the output shaft of a drive motor.

4. Device according to one of the preceding claims, **characterized in that** it comprises an endpiece (15) intended to connect said treatment chamber (1, 1') on the one hand to said switching member (3, 10) and on the other hand to said supply (2) of sterilizing liquid.

5. Device according to Claim 4, **characterized in that** a second flexible connection element (15) connected in a leaktight and removable manner to said endpiece (15) is arranged between said switching member and said treatment chamber (1).

6. Device according to one of the preceding claims, **characterized in that** it comprises a treatment chamber (1') made up of two parts (1'a, 1'b) which are joined to each other in a removable and leaktight manner.

7. Device according to one of Claims 1 to 4, **characterized in that** it comprises a chamber (1'') made up of a tubular element (16), one end of which is open to receive the working part of an endoscope (E), the inside of this chamber (1'') being connected on the one hand to said switching member (3, 10) by way of a joining piece (24) and on the other hand to said supply of sterilizing liquid via the inlet channel (25) for the biopsy forceps of said endoscope (E).

8. Device according to Claim 7, **characterized in that** said tubular chamber (1'') includes a tubular element (16) which is open at both its ends, each of them being engaged in an annular groove (17, 18) of two closure members (21, 22), with interposition of a sealing joint (19, 20).

## Patentansprüche

1. Vorrichtung zum Reinigen und Sterilisieren im Inneren eines Hohlraumes (1) mit einer Zuführ (2) sterilisierender Flüssigkeit zu diesem Hohlraum (1) sowie Mitteln, um im Inneren dieser sterilisierenden Flüssigkeit Veränderungen des Drucks, der Amplitude, Frequenz und Steilheit dieser Veränderungen zu induzieren, die in der Lage sind, im Inneren dieser Flüssigkeit Kavitation hervorzurufen, **dadurch gekennzeichnet, dass** die benannten Mittel, Druckveränderungen zu induzieren, eine Flüssigkeitssäule zwischen dem benannten Hohlraum und einem Schaltglied (3, 10) umfassen, das in der Lage ist, den benannten Hohlraum (1) zyklisch mit einem Unterdruck (7), dessen Wert zu der benannten Amplitude in Beziehung steht, bzw. mit dem Aussendruck in Verbindung zu bringen.

2. Vorrichtung gemäss Anspruch 1, **dadurch gekennzeichnet, dass** sie einen Hauptkanal (4), der das benannte Schaltglied mit dem benannten Behandlungshohlraum (1) verbindet, zwei Kanäle (5, 6), die den benannten Hauptkanal (4) mit dem Aussendruck bzw. mit dem benannten Unterdruck verbinden, wobei das benannte Schaltglied (3, 10 ) Verbindungswege (10b, 10c) zwischen den benannten Kanälen (5, 6) und dem benannten Hauptkanal (4) besitzt und zwischen zumindest zwei Stellungen bewegt werden kann, in deren einer einer der benannten Verbindungswege (10b) den Hauptkanal (4) mit der Atmosphäre in Verbindung bringt und in deren anderer der andere der benannten Verbindungswege (10c) den Hauptkanal mit dem benannten Unterdruck in Verbindung bringt, sowie Antriebsorgane umfasst, um das benannte Schaltglied von der einen in die andere Stellung und umgekehrt zu verschieben.

3. Vorrichtung gemäss Anspruch 2, **dadurch gekennzeichnet, dass** das benannte Schaltglied ein drehbares Glied ist und sich zwangsläufig mit der Ausgangswelle eines Antriebsmotors bewegt.

4. Vorrichtung gemäss einem der vorangehenden Ansprüche, **dadurch gekennzeichnet, dass** sie ein Übergangsstück (15) umfasst, das dazu bestimmt ist, den benannten Behandlungshohlraum (1, 1') einerseits mit dem benannten Schaltglied (3, 10) und andererseits mit der benannten Zufuhr (2) sterilisierender Flüssigkeit zu verbinden.

5. Vorrichtung gemäss Anspruch 4, **dadurch gekennzeichnet, dass** ein zweites, biegsames Verbindungselement (15), das dicht und abnehmbar mit dem benannten Übergangsstück (15) verbunden ist, zwischen dem benannten Schaltglied und dem benannten Behandlungshohlraum (1) angeordnet ist.

6. Vorrichtung gemäss einem der vorangehenden Ansprüche, **dadurch gekennzeichnet, dass** sie einen Behandlungshohlraum (1') umfasst, der aus zwei Teilen (1'a, 1'b) besteht, die abnehmbar und dicht aneinandergefügt sind.

7. Vorrichtung gemäss einem der Ansprüche 1 bis 4, **dadurch gekennzeichnet, dass** sie einen Hohlraum (1") umfasst, der aus einem röhrenförmigen Element (16) besteht, dessen eines Ende offen ist, um den Arbeitsabschnitt eines Endoskops (E) aufzunehmen, wobei das Innere dieses Hohlraumes (1") einerseits über ein Verbindungsstück (24) mit dem benannten Schaltglied (3, 10) und andererseits durch den Eingangskanal (25) der Biopsiezange des benannten Endoskops (E) mit der benannten Zuführ sterilisierender Flüssigkeit verbunden ist.

8. Vorrichtung gemäss Anspruch 7, **dadurch gekennzeichnet, dass** der benannte röhrenförmige Hohlraum (1") ein röhrenförmiges Element (16) umfasst, das an seinen beiden Enden offen ist, deren jedes unter Zwischenschaltung einer Dichtung (19, 20) in eine Ringnut (17, 18) von zwei Verschlussorganen (21, 22) eingreift.
